# EUROPEAN PATENT APPLICATION

(11) **EP 4 789 699 A1**
(43) Date of publication of application: **12.08.2026**
(21) Application number: 26156516.2
(22) Date of filing: 05.02.2026
(51) Int. Cl.: A61M 1/36

(54) **DETERMINATION OF GAS EXCHANGE DEVICE PERFORMANCE**

(30) Priority: 10.02.2025 EP 25156979
(71) Applicant: MAQUET Cardiopulmonary GmbH, 76437 Rastatt (DE)
(72) Inventor: Loderer, Christian, 8500-372 Portimao (PT)
(74) Representative: EIP

(57) **Abstract**

A system (100) is disclosed, comprising a gas exchange device (110) for transferring blood gases between blood and a gas flow passing through the gas exchange device. A flow rate of carbon dioxide V_{g}CO₂ transferred to the gas flow is determined based on a difference between the flow rate of carbon dioxide VₑCO₂ in an exhaust gas and the flow rate of carbon dioxide VₛCO₂ in an inlet gas. A performance of the gas exchange device is determined based on the flow rate of carbon dioxide V_{g}CO₂ transferred to the gas flow and a reference flow rate V_{ref}CO₂, wherein the reference flow rate is based on at least one of a flow rate of blood through the gas exchange device Q_{b}, and a flow rate of the gas flow Qₛ.

## Description

### Technical Field

The present invention relates to methods and systems for gas exchange in a circulatory system of a patient, using a gas exchange device. More particularly, the present disclosure concerns techniques for determining a performance of such a gas exchange device.

### Background

Gas exchange in blood is a physical process by which gases move through a membrane, such as the blood-air barrier in the alveoli of a mammal lung, to allow oxygen to be taken up by the blood and carbon dioxide to be released from the blood.

In case of respiratory failure or bypass, an external gas exchange device may be used to support or replace the function of a patient's lungs. The gas exchange device typically uses a sweep gas to oxygenate the blood and allow carbon dioxide to be released from the blood.

The performance of the gas exchange device is crucial for patient safety and effective medical treatment. Too low uptake of oxygen or too low release of carbon dioxide may quickly lead to dangerous situations in which the health and safety of the patient is put at risk. One commonly monitored parameter is the oxygen uptake in the blood, which may be estimated by a difference in oxygen content of the incoming sweep gas and the oxygen content of the outgoing exhaust gas, i.e., the flow rate of oxygen before and after the gas exchange has taken place. Measuring the oxygen content, especially at the relatively high concentrations typically used for the sweep gas, may however be challenging from a technical point of view and require oxygen sensors that can differentiate small changes in oxygen levels to accurately assess the gas exchange efficiency.

Therefore, there is a need for improved and alternative techniques for monitoring and determining the performance of the gas exchange device.

### Summary

In view of the above, the present disclosure provides an improved or alternative technology having the features set out in the independent claims.

Hence, according to a first aspect, there is provided a method for determining a performance of a gas exchange device, which may be coupled to a circulatory system of a patient to transfer one or more blood gases between blood in the circulatory system and a gas flow passing through the gas exchange device, the gas flow entering the gas exchange device as an inlet gas and exiting the gas exchange device as an outlet gas. The method comprises determining a flow rate of carbon dioxide (CO₂) transferred to the gas flow based on a CO₂ concentration in the exhaust gas, a flow rate of CO₂ in the inlet gas, and a flow rate of the exhaust gas. The performance of the gas exchange device may be determined based on the flow rate of CO₂ transferred to the gas flow and a reference flow rate. The reference flow rate is based on a model describing a flow rate of CO₂ transferred to the gas flow as a function of at least one of: a flow rate of blood through the gas exchange device, a flow rate of the gas flow passing through the gas exchange device, a concentration of CO₂ in the inlet gas, a partial pressure of CO₂ in blood pre-gas exchange, a haemoglobin concentration in the blood, and a pH of the blood. The pH of the blood may refer to the pH of the blood pre-gas exchange or the pH of the blood post-gas exchange.

According to a second aspect, a system is provided, comprising a gas exchange device for transferring blood gases between blood and a gas flow passing through the gas exchange device, the gas flow entering the gas exchange device as an inlet gas and exiting the gas exchange device as an exhaust gas, as well as a CO₂ sensor configured to generate sensor data indicating a CO₂ concentration in the exhaust gas. A control unit is provided to determine a flow rate of CO₂ transferred to the gas flow based on the CO₂ concentration in the exhaust gas, a flow rate of CO₂ in the inlet gas, and a flow rate of the exhaust gas, and to determine a performance of the gas exchange device based on the flow rate of CO₂ transferred to the gas flow and a reference flow rate. The reference flow rate is based on a model describing a flow rate of CO₂ transferred to the gas flow as a function of at least one of: a flow rate of blood through the gas exchange device, a flow rate of the gas flow through the gas exchange device, a concentration of CO₂ in the inlet gas, a partial pressure of CO₂ in blood pre-gas exchange, a haemoglobin concentration in the blood, and a pH of the blood.

According to a third aspect, there is provided a system comprising a gas exchange device for transferring blood gases between blood and a gas flow passing through the gas exchange device, the gas flow entering the gas exchange device as an inlet gas and exiting the gas exchange device as an exhaust gas. The system further comprises a gas supply module configured to generate a first signal indicating a flow rate of CO2 in the inlet gas (if any), and an exhaust gas module configured to generate a second signal indicating a flow rate of CO2 in the exhaust gas. A control unit is provided to receive the first and second signals and to process them to determine a flow rate of CO2 transferred to the gas flow based on difference between the flow rate of CO2 in the exhaust gas and the flow rate of CO2 in the inlet gas. The flow rate of CO2 transferred to the gas flow may be used together with a reference flow rate to determine a performance of the gas exchange device. The reference flow rate may be based on at least one of a flow rate of blood through the gas exchange device, a flow rate of the gas flow through the gas exchange device, a concentration of CO₂ in the inlet gas, a partial pressure of CO₂ in blood pre-gas exchange, a haemoglobin concentration in the blood, and a pH of the blood.

In the above aspects, the flow rate of CO₂ transferred to the gas flow is compared to a reference flow rate, which may be derived from a model, to retrieve information about the performance of the gas exchange device. The reference flow rate may, for example, describe an ideal scenario, such as the expected flow rate of CO₂ transferred by the gas exchange device assuming it is in pristine condition, without wear or deterioration. Thus, the reference flow rate may effectively mirror the performance of a new, or fresh, gas exchange device operating at its full 100% capacity. Comparing the actual CO₂ flow rate from the gas exchange device with the 100% capacity flow rate enables assessment of the device's efficiency. The 100% capacity flow rate may be determined based on one or more of the above-mentioned parameters, such as flow rate of blood through the gas exchange device and/or the flow rate of the gas flow. By comparing the actual CO₂ flow rate against a baseline corresponding to a performance when the gas exchange device was new, it may be possible to quantitatively determine changes in the device's efficiency, such as the extent to which the device's efficiency has declined - or recovered - over time. The efficiency typically reflects the gas transfer capacity of the membrane, which tends to decline over time due to accumulation of blood clots, deposition of a layer increasing diffusion resistance, and water vapour condensing inside, and blocking, fibres of the membrane. A temporary recovery may be observed, should the blood clots dissolve or the condensed water be removed.

Comparing the actual CO₂ flow rate transferred to the gas flow with an expected CO₂ flow rate may not only highlight the degradation or recovery in the device's functional capacity but also aid in evaluating its current operational adequacy relative to its initial performance standards. Further, it is possible to monitor and evaluate trends in the gas exchange rates between the blood and the gas flow. If a monitored parameter falls outside acceptable limits, this indicates the need for replacement or operator intervention.

The reference flow rate may be obtained in various ways, typically as a function of one or more parameters or variables describing the conditions under which the gas exchange device operates. This may be referred to as a model, or mathematical model, which may be designed in various ways. The set of parameters may be combined with a set of constants or weights specific for the design of the gas exchange device model used. As mentioned above, the variables may include one or more of a flow rate of blood through the gas exchange device, a flow rate of the gas passing through the gas exchange device, concentration of CO₂ in the inlet gas, a partial pressure of CO₂ in the blood pre-gas exchange, a haemoglobin concentration in the blood, and pH of the blood. One or more of these variables may be provided as input to the model when determining the reference flow rate of CO₂, reflecting the specific conditions under which the gas exchange device operates.

The constants may be determined based on experimental data, preferably pertaining to gas exchange devices of the same or similar design. The constants may, for example, relate to a permeability of a membrane separating the blood from the sweep gas, indicating how readily a gas such as CO₂ and O₂ can pass through the membrane. The constants may also relate to an effective area of the membrane, over which the gas exchange occurs, as well as a flow profile of the blood passing through the device. In further examples, the constants may pertain to operational parameters affecting the gas exchange efficiency, such as heating of the membrane or the sweep gas.

The reference CO₂ flow rate may hence be calculated based on the type of gas exchange device and one or more operational parameters of the gas exchange device, where the type of gas exchange device determines which model (or constants) to use, and the operational parameter(s) serve as input to the model. In some examples, the reference CO₂ flow rate is determined based on a combination of at least two parameters, such as the flow rate of blood through the gas exchange device (also referred to as 'blood flow') and the flow rate of the gas flow supplied to the gas exchange device (also referred to as 'sweep gas flow'). Alternatively, or additionally, the reference CO₂ flow rate may be determined based on the blood flow and the partial pressure of CO₂ in the blood pre-gas exchange, and/or the flow rate of blood and the haemoglobin concentration in the blood. Further combinations and specific examples of models are discussed in the detailed description of the drawings.

It should be understood that the model describing the relationship between the reference flow rate and the parameters, such as one or more of the flow rate of blood through the gas exchange device, the flow rate of the gas flow through the gas exchange device, the concentration of CO₂ in the inlet gas, the partial pressure of CO₂ in blood pre-gas exchange, the haemoglobin concentration in the blood, or the pH of the blood, may comprise or be implemented as a data structure, such as a lookup table, dataset, or other repository of reference values. In such cases, one or more of the parameters described above may be used as keys or indices to retrieve a corresponding reference flow rate from the data structure. Thus, the reference flow rate may in some examples be obtained by data retrieval rather than by calculation.

In an example, the reference flow rate is determined based on the flow rate of blood through the gas exchange device. In another example, the reference flow rate is determined based on the flow rate of the gas flow, or a combination of the flow rate of blood and the gas flow through the gas exchange device.

The exhaust gas module may comprise a CO₂ sensor for generating sensor data indicating a CO₂ concentration in the exhaust gas. This concentration may be used together with the total flow rate of the exhaust gas to determine the flow rate of CO₂ in the exhaust gas. The exhaust gas module may be configured to determine the total flow rate of the exhaust gas, using direct or indirect measurements as will be outlined in the following.

The total flow rate of the exhaust gas may be challenging to determine. A direct flow rate measurement at an outlet of the gas exchange device risks missing leakages, such as gas escaping through an emergency outlet which is commonly implemented in many gas exchange devices. Further, the outgoing gas flow tends to be saturated with water vapour, which may condensate and thereby limit the accuracy of the flow rate measurements. In some examples, it is therefore proposed to determine the flow rate based on a mass equilibrium approach of oxygen, in which the amount of oxygen leaving the gas exchange device is assumed to correspond to the difference between the amount of oxygen supplied to the gas exchange device and the amount of oxygen taken up by the blood during the gas exchange. The amount of oxygen taken up by the blood may be determined by comparing the oxygen content in the blood post-gas exchange with the oxygen content in the blood pre-gas exchange based on blood sensor data. By subtracting the amount of oxygen that is taken up by the blood from the amount of oxygen that is supplied to the gas exchange device, the amount of oxygen that remains in the gas flow after the gas exchange can be determined. By combining the amount of oxygen in the exhaust gas with the concentration of oxygen in the exhaust gas, the total flow rate of exhaust gas can be determined without directly measuring the flow rate using, for example, a flow meter.

Hence, according to an example, determining the flow rate of CO₂ in the exhaust gas may comprise:
determining the oxygen uptake in the blood based on the flow rate of blood and a difference between an oxygen concentration in the blood exiting the gas exchange device and the oxygen concentration in the blood entering the gas exchange device,
determining the flow rate of oxygen in the exhaust gas based on a difference between a flow rate of oxygen in the inlet gas and the oxygen uptake in the blood, and
determining the flow rate of the exhaust gas based on the flow rate of oxygen in the exhaust gas and the oxygen concentration in the exhaust gas.

It should be noted, however, that there are alternative methods for determining the flow rate of the exhaust gas. In some examples, the flow rate can be measured using a flow sensor placed at the gas outlet, or integrated into the gas exchange device, or arranged further away from the gas exchange device, connected to the same by a tube. In some examples, the flow rate can be measured by determining the inlet gas flow rate and adjusting for humidity uptake in the gas exchange device. These methods may be useful for verifying the flow rates obtained through the equilibrium approach, serving as effective sanity checks.

Typically, CO₂ flow rate from the gas exchange device is determined by measuring the CO₂ concentration in the exhaust gas and multiplying it by the total flow rate of the exhaust gas. This method may be referred to as exhaust gas measurements. However, an alternative or complementary method involves blood gas measurements. This method determines the CO₂ flow rate by comparing the CO₂ content in the blood before and after gas exchange. Specifically, it involves calculating the difference between the CO₂ content in the blood entering the gas exchange device and the blood leaving the gas exchange device, and then multiplying this difference by the blood flow rate. Blood gas measurements can either serve as an independent method or complement exhaust gas measurements to verify their accuracy and may be performed intermittently or continuously.

Hence, in some instances, the flow rate of CO₂ released or transferred to the gas flow, as calculated from blood gas measurements (i.e., the 'blood gas-based flow rate'), may be compared with the CO₂ flow rate transferred to the gas flow, as determined from exhaust gas measurements (i.e., the 'gas flow-based flow rate'), to serve as a 'sanity check' for the accuracy of the exhaust gas measurements. Although exhaust gas measurements are generally considered more accurate than the blood gas measurements, significant discrepancies between the two methods may signal an issue. The blood gas-based CO₂ flow rate and the gas flow-based CO₂ flow rate may be compared to each other, for example by studying the difference or quotient between the two. If the difference or quotient exceeds a predefined threshold, an error state may be identified. This error state may trigger an alert, issuing a warning or error signal, which may necessitate operator intervention.

It will be appreciated that that CO₂ can be transported in the blood in three primary forms: dissolved CO₂, hydrogen carbonate ( ${HCO}_{3}^{-}$), and carbamino compounds. Typically, about 30% of all CO₂ is transported as carbamino compounds, 60% through production of ${HCO}_{3}^{-}$ ions in the red blood cells, and about 10% as dissolved in plasma. During the blood-gas exchange in the lungs, ${HCO}_{3}^{-}$ ions play an important role. As blood reaches the lungs, HC03 ions are converted into CO₂ which may be exhaled from the body through respiration. In the context of the present disclosure, the term 'CO₂ concentration' typically refers to the total concentration of CO₂ present in the blood, i.e., all three primary forms mentioned above, whereas the term 'partial pressure of CO₂' typically refers to the concentration of dissolved CO₂. Conventionally, the partial pressure of CO₂ may be used in clinical practise to assess the gas exchange efficiency.

In the above examples, some parameters for determining CO₂ flow rate transferred to the gas flow may be predetermined or known from system settings, whereas other parameters may be retrieved from sensor data. The flow of blood through the gas exchange device may be measured by means of an ultrasonic flow sensor, or be a known parameter determined by the settings of the system, such as the pump rate of a pump used for circulating the blood through the system. Further, the oxygen concentration in the inlet gas as well as the total flow of the inlet gas may be known from the settings of the inlet gas supply, such as a gas blender used to supply the gas exchange device with the required sweep gas.

In some examples, the gas supply module is configured to provide information pertaining to the CO₂ flow rate in the inlet gas. The gas supply module may, for example, comprise a gas blender or mixer that is operable to deliver a specific concentration of oxygen and/or CO₂ at a specific flow rate. The concentration and flow rate of oxygen and/or CO₂ may hence be known, or predetermined, by operational settings of the gas supply module and used as input to the control unit when determining the performance of the gas exchange device, as discussed above. In further examples, the gas supply module comprises one or more sensors, such as a flow sensor, an oxygen concentration sensor, or a CO₂ concentration sensor, for generating information that can be used to determine the CO₂ flow rate in the inlet gas.

The oxygen and/or CO₂ content in the blood post-gas exchange and the blood pre-gas exchange, as well as the oxygen and/or CO₂ concentration in the exhaust gas, may however be retrieved from sensor data. It will hence be appreciated that various sensors may be provided to measure the oxygen and/or CO₂ content, such as partial pressure, depending on the system's specific configuration. In some configurations, one or more of these sensors may be integrated directly within the gas exchange device, while in others, they could be separate components designed to connect to the gas exchange device. Each of these sensors may be communicatively linked to the control unit, transmitting sensor signals for processing. The control unit may utilise these signals for the calculations and determinations discussed, and to input data into the model predicting the reference CO₂ flow rate.

In the context of the present disclosure, the term 'flow' or 'flow rate' typically refers to how a gas or blood is moved through the system. The flow rate may either refer to the total flow rate of gas supplied to the gas exchange device or exhausted by the gas exchange device, or the flow rate of a particular component of the gas, such as O₂ or CO₂. The flow rate may generally be understood as a term describing the quantity of a fluid moving through a given cross-sectional area per unit time and can be measured in terms of either volume or mass. The former may be referred to as a volumetric flow rate and describes how much volume of a fluid passes through a given area in a specific time. In the SI system, the volumetric flow rate is often expressed in cubic metres per second (m³/s). However, in the present context the flow rate may commonly be expressed in litres per minute (L/min). Thus, the volumetric flow rate typically describes how many litres of fluid, such as oxygen or blood, are being supplied to (or exhausted by) the gas exchange device per minute. Alternatively, the flow rate may be measured as a mass flow rate, describing the mass of a fluid (such as oxygen or blood) passing through a given area in a specific time. The mass flow rate may thus be expressed in kilograms per second (kg/s). Mass flow rate may be particularly useful when the density of the gas might change under varying conditions, as the mass flow rate may provide a measure that is independent of temperature and pressure. It will be appreciated the volumetric flow rate is proportional to the mass flow rate, and that the volumetric flow rate therefore be converted into a mass flow rate (and vice versa) for a given temperature and pressure of the gas.

The standard cubic centimetre per minute (SCCM) is an example of a unit that can be used to quantify the flow rate of a fluid and may be understood as the mass flow rate of one cubic centimetre per minute of a gas at a density defined at some standard conditions for temperature and pressure. The standard conditions may vary between different regulatory bodies but may in an example correspond to a temperature of 0°C and a pressure of 1.013 bar.

Further, for the discussion of oxygen content in blood, it should be noted that the oxygen content in the blood typically refers to the total concentration of oxygen carried by the blood flow. Oxygen typically exists in the blood in two major forms: bound to haemoglobin and dissolved directly in the plasma. It will be appreciated that other forms may exist, but that these two tend to account for most of the oxygen content. Commonly, a major part of the oxygen transported in the blood is bound to haemoglobin (commonly referred to as 'saturation'), whereas a smaller fraction of the oxygen in the blood is dissolved in the plasma (commonly referred to as 'partial pressure'). Hence, the oxygen concentration typically refers to the total concentration of oxygen as described by the oxygen saturation, haemoglobin concentration and the partial pressure of oxygen as well as constants. The oxygen concentration may be expressed as a volume of oxygen per unit volume of blood (such as millilitres of oxygen per decilitre of blood) or amount of substance of oxygen per unit volume of blood (such as millimoles of oxygen per decilitre of blood).

The oxygen concentration, or partial pressure, in the inlet gas and the exhaust gas may typically be measured as a percentage of the total gas mixture. For example, medical oxygen supplied to the gas exchange device may have concentrations from around 21% (corresponding to the concentration in air) to 100%, depending on the clinical need. The percentage typically describes a volume ratio, like litres of oxygen per litre of total gas. However, in some examples the concentration may also be expressed as a mole fraction (number of moles of oxygen per mole of gas mixture) or an amount of substance oxygen per unit volume of gas mixture.

The blood pre-gas exchange typically refers to blood that has circulated through the body and has delivered oxygen to the tissue. As a result, it may have a higher concentration of carbon dioxide. The blood pre-gas exchange may also be referred to as pre-oxygenation blood, deoxygenated blood or venous blood. The oxygen-poor blood may be supplied to the gas exchange device for enrichment with oxygen and/or removal of carbon dioxide.

The blood post-gas exchange, also referred to as oxygen-rich blood, oxygenated blood or arterial blood, is understood as blood that has been subject to gas exchange in the gas exchange device, in which oxygen may be supplied to the blood and/or carbon dioxide be removed from the same. The oxygen-rich blood may be returned to the patient's body to supply the tissues with oxygen.

As mentioned above, the oxygen content in the blood may refer to the total concentration of oxygen carried by the blood flow, as described by the saturation, haemoglobin concentration, the partial pressure of oxygen, and constants. Thus, the sensor data may indicate both - i.e., the saturation in the oxygen-rich blood and in the oxygen-poor blood, as well as the partial pressure of oxygen in the oxygen rich-blood and in the oxygen-poor blood. By taking the saturation, the haemoglobin concentration, and the partial pressure into account, the total concentration or amount of oxygen taken up by the blood during the gas exchange may be determined at a relatively high accuracy.

Alternatively, or additionally, for oxygen-poor blood, or blood which is not fully saturated with oxygen, the saturation can be determined based on the partial pressure of oxygen. This is based on the establishment of a known relationship between the partial pressure of oxygen and the saturation. Thus, by measuring the partial pressure of oxygen in the oxygen-poor blood, the relationship may be used to estimate the oxygen saturation without directly measuring the oxygen saturation level. It should however be noted that the relationship between saturation and the partial pressure of oxygen may be influenced by other parameters, such as pH, partial pressure of carbon dioxide, and the level of 2,3-DPG. These parameters may be taken into account to further improve the estimation of the saturation level.

Further features and advantages of the invention will become apparent from the following description of preferred embodiments of the invention, given by way of example only, which is made with reference to the accompanying drawings.

### Brief Description of the Drawings

The above, as well as additional objects, features, and advantages of the present disclosure will be better understood through the following illustrative and non-limiting detailed description of examples, with reference to the appended drawings. Same reference numerals will be used for similar elements.
Figure 1 shows a system according to an example, comprising a gas exchange device coupled to an extracorporeal circuit.
Figure 2 shows a system according to an example, illustrating various sensors employed to retrieve observational data from the system.
Figure 3 is a flowchart illustrating an example of a method for determining a performance of a gas exchange device.
Figure 4 is a flowchart illustrating another example of such a method, in which the total flow rate of exhaust gas is determined.

As illustrated in the figures, the sizes of the elements and features may be exaggerated for illustrative purposes and, thus, are provided to illustrate the general structures of the examples.

### Detailed Description

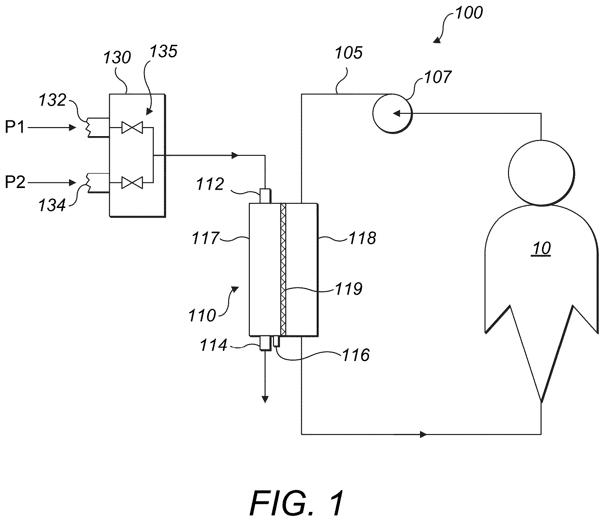

Figure 1 shows by way of example a system 100 for exchanging one or more gases with blood in a circulatory system of a patient 10. The system 100 comprises a gas exchange device 110 for transferring blood gases between blood and a gas flow, wherein the blood is circulated in an extracorporeal circuit 105 and the gas flow is entering the gas exchange device 110 as an inlet gas via an inlet 112 and exiting the gas exchange device 110 as an exhaust gas via an outlet 114. The gas flow supplied to the gas exchange device 110 may also be referred to as a sweep gas.

Examples of systems 100 according to the present disclosure include cardiopulmonary bypass machines (heart-lung machines) providing circulatory and respiratory support in the operating theatre while the heart is stopped for surgery. Further examples include extracorporeal membrane oxygenator (ECMO) devices providing circulatory and respiratory support in intensive care units, and extracorporeal CO₂ removal (ECCO2R) devices removing excess carbon dioxide from the blood.

The gas exchange device 110 is configured to facilitate exchange of gases between the patient's blood and a gas flow passing through the gas exchange device 110. Typically, the gas exchange device 110 is configured to allow oxygen to pass from the gas flow into the blood, thereby oxygenating the blood, and to allow CO₂ to be released from the blood and pass into the gas flow. It will be appreciated that other types of gases and substances may be exchanged in a similar way, including anaesthetic agents and nitric oxide.

The gas exchange device 110, which in some examples may be referred to as an oxygenator, may comprise a gas region 117 and a blood region 118 separated by a gas-permeable membrane 119. The blood in the extracorporeal circuit 105 may be circulated through the blood region 118 by a circulation unit 107, whereas the gas flow may flow through the gas region 117 via the gas inlet 112 and the gas outlet 114. Due to a partial pressure gradient, or a concentration difference, between individual components of the gas flow and the corresponding component in the blood, this component may be passed through the membrane 119 from the gas region 117 into the blood region 118 or vice versa. In particular, oxygen may be passed from the gas region 117 to the blood region 118 through the membrane 119 so that the oxygen-poor blood is oxygenated into oxygen-rich blood. Vice versa, CO₂ may be passed from the blood region 118 to the gas region 117 through the membrane 119 so that CO₂ is removed from the flow of blood. This may be referred to as a flow rate of CO₂ being transferred to the gas flow.

Several types of membranes 119 may be employed. In an example, the gas exchange device 110 comprises a hollow fibre membrane, in which the blood flows in the blood region 118 inside the fibres and a gas mixture flows in the gas region 117 outside the fibres. Oxygen and CO₂ exchange may occur across the membrane formed by the walls of the hollow fibres. In the example illustrated in figure 1, however, the membrane 119 is schematically illustrated as a sheet.

The circulation unit 107 may, for example, comprise a roller pump or a centrifugal pump, depending on the specific type of system 100. Roller pumps typically comprise a rotating roller compressing a flexible tube or membrane. As the roller rotates, is squeezes the tube, creating a pulsatile flow of blood. Centrifugal pumps, on the other hand, use a rapidly rotating impeller to create a centrifugal force which pushes the blood outwards to generate a substantially continuous blood flow. Centrifugal pumps have been observed to reduce the risk for haemolysis, i.e., breakdown of red blood cells, and improve patient comfort.

The transfer capacity of oxygen or CO₂, i.e., the amount of oxygen or CO₂ which is supplied to or removed from the flow of blood per unit of time depends, inter alia, on the flow rate of the blood, the flow rate of the gas flow, the composition of the gas flow, and the state of the gas exchange device 110 itself. In particular, the gas transfer capacity of the membrane 119 can decline over time due to accumulation of blood clots, deposition of a layer increasing diffusion resistance, and water vapour condensing inside, and blocking, the hollow fibres of the membrane 119. A gas exchange device 110 incapable of oxygenating the blood to a satisfying degree may hence be considered to be in a degraded or error state.

The gas flow may be provided by a gas supply module, for example comprising a gas blender 130. Typically, the gas blender 130 comprises a plurality of inlets 132, 134 configured to receive medical gas from a respective gas source (not shown). In the example shown in figure 1, the gas blender 130 comprises a first inlet 132 for a medical oxygen flow P1 and a second inlet 134 for a medical air flow P2. It will however be appreciated that more inlets may be provided, and that the gas blender 130 may be used for supplying other types of gas, such as carbon dioxide, nitric oxide or anaesthetic agents.

The medical oxygen may be supplied to the gas blender 130 from an oxygen source, which may be an oxygen outlet of a central medical gas system of a hospital facility, a pressurised gas cylinder, or any other type of oxygen source suitable for delivering oxygen for medical applications. The same applies to the air, which may be supplied from a pressurised air outlet of a central medical gas system of a hospital facility, a pressurised cylinder, or retrieved and pressurised from the surrounding air.

The gases received at the inlets 132, 134 may be mixed into a sweep gas mixture by a valve arrangement (collectively indicated by reference numeral 135) and supplied to the gas inlet 112 of the gas exchange device 110 as an inlet gas. The valve arrangement 135 may be configured to adjust the oxygen concentration and flow rate of the inlet gas according to the patient's needs, allowing the healthcare professionals to control the oxygen levels delivered to the patient. The valve arrangement 135 may be electronically controlled, and in some examples configured to utilise sensor input to achieve the accurate gas composition and flow rate.

The gas blender 130 may thus be operated to deliver a specific concentration of oxygen, such as 100% oxygen, at a specific flow rate, such as 4 litres per minute. The concentration and flow rate of oxygen may hence be known, or predetermined, by the operational settings of the gas blender 130, and used as input when determining the performance of the gas exchange device 110, as discussed below.

In the example shown in figure 1, an emergency outlet 116 is provided at the gas region 117 of the gas exchange device 110. The emergency outlet 116 forms an additional outlet, i.e., in addition to the 'primary' outlet 114 discussed above, and serves to protect the gas exchange device 110 and the patient 10 from damage due to overpressure, should the primary outlet 114 be obstructed or blocked. The emergency outlet 116 may be arranged in a constant-open state, allowing exhaust gas to pass freely therethrough during operation of the gas exchange device 110. Hence, the total flow of exhaust gas may be divided in partial flows: a first partial flow passing through the gas outlet 114, and a second partial flow passing through the emergency outlet 116.

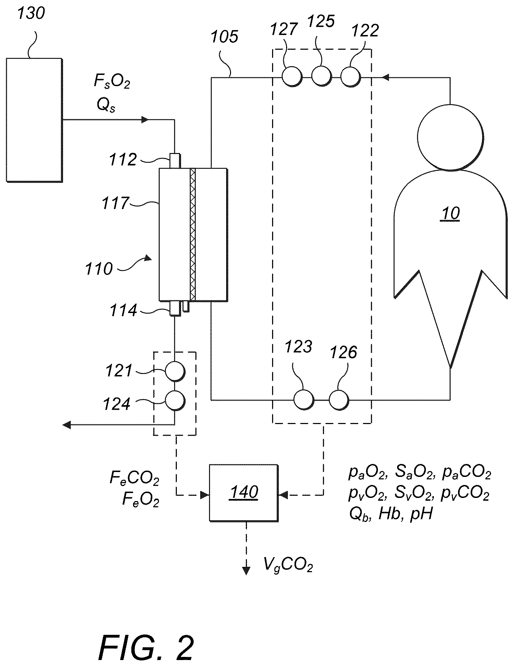

One or more sensors may be provided to generate sensor data indicating, for example, a characteristic of the blood, such as flow rate of blood Q_{b} through the gas exchange device 110, a haemoglobin concentration Hb, oxygen saturation, or pH in the blood, and blood gases such as CO₂ and oxygen pre- and post-gas exchange. Further, one or more sensors may be arranged to measure a characteristic of the gas flow, i.e., the inlet gas and/or the outlet gas. One or more of these sensors may be implemented in a gas supply module as discussed above, and/or in an exhaust gas module configured to generate a signal indicating a CO₂ flow rate in the exhaust gas. Examples of measured characteristics of the gas flow may include flow rate Qₛ of the gas flow, and partial pressure of CO₂ as well as oxygen concentration pre- and post-gas exchange, pressure, temperature, and moisture level. A few examples of such sensors, as well as the use of the data generated by the sensors, will be discussed in the following with reference to figures 2-4.

Figure 2 shows by way of example a system 100, which may be similarly configured as the system 100 in figure 1. Hence, during operation of the system 100, oxygen-poor blood of the extracorporeal circuit 105 is supplied to the blood region 118 of the gas exchange device 110, where it is oxygenated by the gas flow passing through the gas region 117 of the gas exchange device 110.

The efficiency of the gas exchange device 110 in oxygenating blood and removing CO₂ is vital for patient safety and the efficacy of medical treatments. Insufficient oxygen uptake or inadequate CO₂ removal can rapidly result in hazardous conditions, jeopardizing the patient's health and safety.

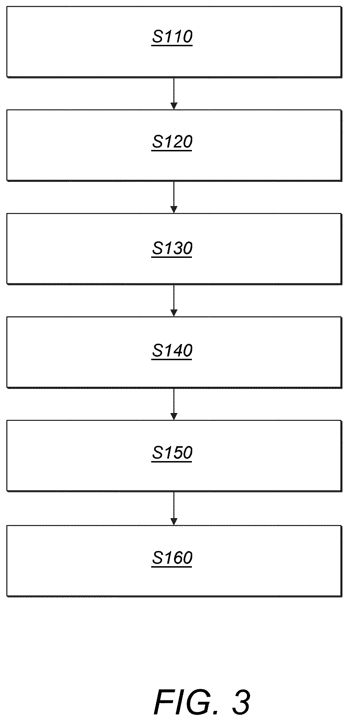

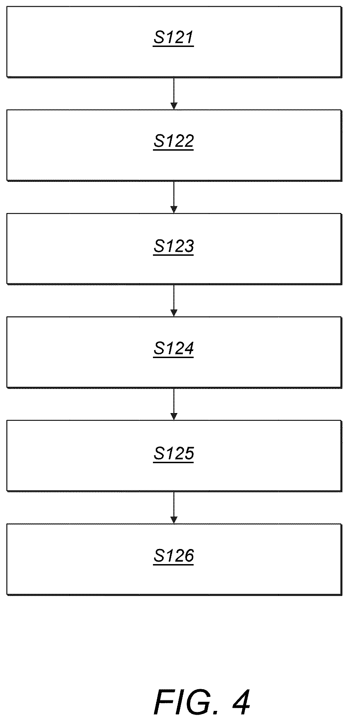

Therefore, sensors may be provided for monitoring various characteristics of the system. Their outputs may be relayed to a control unit 140, which may be configured to analyse the data to assess the gas exchange device's 110 efficiency in removing CO₂ from the patient's blood. The efficiency, or performance, may be determined by calculating the CO₂ flow rate transferred to the gas flow and comparing this against a reference flow rate derived from a model. Examples of such a method are described in further detail with reference to figures 3 and 4.

The exemplary system 100 in figure 2 comprises a CO₂ sensor 121 and an oxygen sensor 124 arranged to measure a CO₂ concentration FₑCO₂ and an oxygen concentration FₑO₂, respectively, in the exhaust gas leaving the gas exchange device 110 at the gas outlet 114. The CO₂ sensor 121 and the oxygen sensor 124 may, for example, be included in an exhaust gas module as discussed above. Additional or alternative sensors may however be provided, either separately or included in a gas supply module or an exhaust gas module. The system 100 may, for example, comprise a flow sensor for measuring the flow rate Qₛ of sweep gas supplied to the inlet 112 of the gas exchange device 110, a flow sensor for measuring the flow rate Qₑ of exhaust gas leaving the gas exchange device through the outlet 114, as well as an oxygen sensor for measuring an oxygen concentration FₛO₂ of the sweep gas supplied to the gas exchange device 110.

The system 100 further comprises one or more sensors for measuring characteristics, such as blood gases and flow rates, of the blood circulating through the gas exchange device 110. In the present example, an oxygen sensor 122 is arranged to measure an oxygen saturation SᵥO₂ and/or partial pressure of oxygen pᵥO₂ in blood supplied to the gas exchange device 110 (additionally, or alternatively, a sensor may be provided to measure oxygen saturation SₐO₂ in blood leaving the gas exchange device 110), a CO₂ sensor 125 arranged to measure a partial pressure of carbon dioxide pᵥCO₂ in the blood supplied to the gas exchange device, and a flow sensor 127 for measuring a flow rate Q_{b} of the blood. In further examples, sensors may be provided to measure the haemoglobin concentration Hb, and/or pH of the blood supplied to the gas exchange device.

One or more sensors may also be provided to measure characteristics of blood post-gas exchange, such as one or more sensors 123 for measuring an oxygen saturation SₐO₂, a partial pressure of oxygen pₐO₂, or haemoglobin concentration Hb, in blood leaving the gas exchange device 110. There may also be provided one or more sensors 126 for measuring a partial pressure carbon dioxide pₐCO₂ in said blood. As mentioned above, further sensors may be employed depending on the configuration of the system 100, such as a sensor for measuring a flow rate Q_{b} of the blood, as well as pH.

It should be noted that the system shown in figure 2 is merely an example of a possible implementation of the inventive concept, and that various sensor configurations may be employed depending on the specific configuration of the system 100. Furthermore, two or more of the sensor functionalities mentioned above may be provided by a single sensor device, such as oxygen saturation SₐO₂ / SᵥO₂ and haemoglobin concentration Hb.

The sensor data may be supplied to the control unit 140 for determining the performance of the gas exchange device 110. An example of such a method is illustrated in the flow chart of figure 3, which may be studied together with figure 2 for a better understanding of the technology disclosed herein. According to figure 3, the CO₂ flow rate VₑCO₂ in the exhaust gas may be determined based on a CO₂ concentration FₑCO₂ in the exhaust gas, measured by the CO₂ sensor 121, and a flow rate Qₑ of the exhaust gas. A signal indicating the flow rate VₑCO₂ in the exhaust gas may be generated by an exhaust gas module and transmitted to the control unit 140. In case the inlet gas also comprises CO₂, the difference in flow rate VₑCO₂ of CO₂ in the exhaust gas flow and flow rate VₛCO₂ of CO₂ in the inlet gas may be determined to retrieve the flow rate V_{g}CO₂ transferred in the gas exchange. The performance of the gas exchange device 110 may then be determined by comparing the CO₂ flow rate V_{g}CO₂ with a reference flow rate V_{ref}CO₂ retrieved from a model describing an ideal scenario, such as the expected CO₂ flow rate of the gas exchange device during similar operational conditions, as defined by one or more parameters such as flow rate of blood Q_{b}, flow rate of the gas flow Qₛ, CO₂ concentration FₛCO₂ in the inlet gas, etc., assuming the gas exchange device is in pristine condition, without wear or deterioration.

Thus, according to the example shown in figure 3, one step of the method may be to determine S110 the CO₂ concentration in the exhaust gas FₑCO₂. This information may, for example, be retrieved from the CO₂ sensor 121 in figure 2. A further step may be to determine S120 the flow rate Qₑ of the exhaust gas and use this information to calculate S130 the CO₂ flow rate VₑCO₂ in the exhaust gas. There are various ways to determine the flow rate Qₑ of the exhaust gas. One possible example is discussed in greater detail with reference to figure 4. Further, the CO₂ flow rate V_{g}CO₂ transferred to the gas flow during the gas exchange may be determined S140 by subtracting the CO₂ flow rate of the inlet gas VₛCO₂ from CO₂ flow rate VₑCO₂ in the exhaust gas (see Eq. 5 below). In case the inlet gas is void of CO₂, the CO₂ flow rate V_{g}CO₂ transferred to the gas flow equals the CO₂ flow rate VₑCO₂ in the exhaust gas. Optionally, the method may comprise a step of determining the CO₂ flow rate of the inlet gas VₛCO₂. This may include, for instance, sensor measurements or retrieving gas composition information from the gas blender 130.

Thereafter, the CO₂ reference flow rate V_{ref}CO₂ is determined S150 based on a model and compared with the CO₂ flow rate V_{g}CO₂ to determine S160 the performance of the gas exchange device 110.

The model typically includes one or more of a set of parameters or variables describing the conditions under which the gas exchange device operates, such as flow rate Q_{b} of blood through the gas exchange device 110, flow rate of the gas flow Qₛ passing through the gas exchange device 110, partial pressure of CO₂ (pᵥCO₂) in oxygen-poor blood entering the gas exchange device 110, concentration of CO₂ (FₛCO₂) in the inlet gas, a haemoglobin concentration Hb in the blood, and optionally pH of the blood. Further, the model may include a set of constants or weights specific for the design of the gas exchange device model used.

In some examples, a blood gas-based CO₂ flow rate of V_{b}CO₂ released to the sweep gas through the membrane of the gas exchange device 110 may be determined S130 based on the flow rate of blood Q_{b} and a difference in a carbon dioxide concentration CᵥCO₂ in the oxygen-poor blood and a carbon dioxide concentration CₐCO₂ in the oxygen-rich blood: ${V}_{b} {CO}_{2} = {Q}_{b} \left({C}_{v}{CO}_{2}-{C}_{a}{CO}_{2}\right)$ The control unit may retrieve the flow rate of blood Q_{b} from operational settings of a pump circulating the blood in the extracorporeal circuit, and/or by means of a flow sensor arranged to generate a sensor signal indicating the flow rate Q_{b}. It should be noted that the CO₂ concentrations CᵥCO₂ / CₐCO₂ in the blood may be determined in various ways known in the art. One possible approach would be to use a model based on pH and partial pressure pᵥCO₂ / pₐCO₂.

The blood gas-based CO₂ flow rate V_{b}CO₂ released to the sweep gas during the gas exchange may be compared with the gas flow-based CO₂ flow rate V_{g}CO₂ determined by measuring the CO₂ flow rate V_{g}CO₂ in the exhaust gas (and, if applicable, the flow CO₂ flow rate in the inlet gas) to verify the accuracy of the measured CO₂ flow rate V_{g}CO₂. Hence, it may be determined that the blood gas-based CO₂ flow rate V_{b}CO₂ released during the gas exchange corresponds to the gas flow-based CO₂ flow rate V_{g}CO₂ measured in the exhaust gas. Should a difference or quotient between the two flow rates (V_{b}CO₂ and V_{g}CO₂) exceed a predetermined limit, such as 30% or more, an error state may be determined.

As mentioned above, the CO₂ flow rate V_{g}CO₂ transferred to the gas flow may be determined based on the CO₂ concentration FₑCO₂ in the exhaust gas multiplied by the total flow rate Qₑ of the exhaust gas, and by accounting for any CO₂ present in the inlet gas. However, as the total flow rate Qₑ of exhaust gas may be challenging to determine with sufficient accuracy due to leakage flows through, for example, the emergency outlet 116, the flow rate Qₑ of the exhaust gas may instead be calculated based on a mass equilibrium approach of oxygen.

An example of such a method is outlined in figure 4. In this approach, the oxygen uptake in the blood is determined based on the oxygen concentration in blood pre- and post-gas exchange and used together with the oxygen concentration in the exhaust gas to determine the flow rate of the exhaust gas. The method hence comprises a step of determining S121 the oxygen concentration CᵥO₂ in the blood pre-gas exchange, as well as a step of determining S122 the oxygen concentration CₐO₂ in the blood post-gas exchange. The oxygen concentrations pre- and post-gas exchange CᵥO₂, CₐO₂ may be determined based on sensor data indicative of an oxygen saturation SᵥO₂, SₐO₂, a partial pressure pᵥO₂, pₐO₂ of oxygen in the blood pre- and post-gas exchange, respectively, as well as haemoglobin concentration Hb. The haemoglobin concentration Hb may, for example, be measured by a probe that is integrated in the gas exchange device, or via a blood sample. By combining the difference in oxygen concentration pre-gas exchange CᵥO₂ and post-gas exchange CₐO₂ with the flow rate Q_{b} of the blood, the oxygen uptake in the gas exchange V_{b}O₂ may be determined S123: ${V}_{b} {O}_{2} = {Q}_{b} \left({C}_{a}{O}_{2}-{C}_{v}{O}_{2}\right)$ wherein the flow rate Q_{b} of blood may be measured by the flow sensor 127 or retrieved from the settings of the blood pump, such as speed (RPM) and pressure, as well as hematocrit and/or hemoglobin concentratrion Hb, and temperature.

The method further comprises determining S124 a flow rate of oxygen VₛO₂ in the inlet gas, for example based on the oxygen concentration FₛO₂ and the total flow rate Qₛ of the inlet gas. These parameters may be known from the settings of the gas blender 130, and may be provided to the control unit by the gas supply module. The gas blender 130 may, for example, be configured to deliver a total inlet flow of 4 L/min and an oxygen concentration of 80% or 100% oxygen. It will however be appreciated that the total inlet flow and/or the oxygen concentration in some examples may be determined by one or more sensors, such as a flow meter or a sensor for measuring oxygen concentration, which may be included in the gas supply arrangement. Further sensors may be employed to compensate for sensor inaccuracies and other sources or errors. Examples include pressure sensors, temperature sensors, and humidity sensors. The equilibrium approach is based on an assumption that the amount of oxygen leaving the gas exchange device 110 corresponds to the difference between the amount of oxygen supplied to the gas exchange device 110 and the amount of oxygen taken up by the blood during the gas exchange. This mass equilibrium approach can be described by the following expression: ${V}_{g} {O}_{2} = {F}_{s} {O}_{2} ⋅ {Q}_{s} - {F}_{e} {O}_{2} ⋅ {Q}_{e}$ where V_{g}O₂ is the gas flow-based oxygen uptake in the blood (corresponding to the blood gas-based uptake V_{b}O₂ determined in equation 2), FₛO₂ the concentration of oxygen in the sweep gas provided to the gas exchange device 110, Qₛ the flow rate of the inlet gas, FₑO₂ the concentration of oxygen in the exhaust gas, and Qₑ the total flow rate of the exhaust gas. Solving for Qₑ gives: ${Q}_{e} = \frac{{F}_{s} {O}_{2} ⋅ {Q}_{s} - {V}_{g} {O}_{2}}{{F}_{e} {O}_{2}}$ The oxygen concentration *FₑO₂* in the exhaust gas can be determined S125 based on sensor data from the oxygen sensor 124 arranged at the outlet 114 of the gas exchange device 110. The oxygen concentration may then be used together with equation 4 above to determine S126 the total flow of the exhaust gas.

Once the flow rate Qₑ of the exhaust gas is determined, the CO₂ flow rate VₑCO₂ in the exhaust gas may be determined S126 as: ${V}_{e} {CO}_{2} = {Q}_{e} ⋅ {F}_{e} {CO}_{2}$ where FₑCO₂ may be retrieved from the CO₂ sensor 121 as mentioned above.

The CO₂ flow rate V_{g}CO₂ transferred to the gas flow during the gas exchange may be determined as: ${V}_{g} {CO}_{2} = {V}_{e} {CO}_{2} - {V}_{s} {CO}_{2}$ where VₛCO₂ is the CO₂ flow rate of the inlet gas. In case the inlet gas is void of CO₂, the CO₂ flow rate V_{g}CO₂ transferred to the gas flow equals the CO₂ flow rate VₑCO₂ in the exhaust gas.

As previously mentioned, oxygen typically exists in the blood in two major forms: bound to haemoglobin ('oxygen saturation') and dissolved directly in the plasma ('partial pressure of oxygen'). Commonly, most of the oxygen transported by the blood is bound to haemoglobin, whereas a smaller fraction (such as 1.5-3%) is dissolved in the plasma. Hence, the oxygen concentration CᵥO₂ / CₐO₂ typically refers to the combination of oxygen saturation SᵥO₂ / SₐO₂, haemoglobin Hb, and partial pressure pᵥO₂ / pₐO₂. However, as indicated above, the oxygen concentration CᵥO₂ / CₐO₂ may in some examples be approximated by the oxygen saturation SᵥO₂ / SₐO₂ only. In the following, examples of how to determine the respective concentrations will be discussed.

The oxygen saturation SᵥO₂ / SₐO₂ can be understood as the ratio of the available oxygen binding sites of the haemoglobin that are occupied by oxygen molecules. In a healthy adult individual, the oxygen saturation of oxygen-rich blood is usually between 95% and 100%, while in oxygen-poor blood it usually is between 60% and 80%.

Haemoglobin is known to be able to carry about 1.34 mL of oxygen per gram when fully saturated. The concentration of haemoglobin Hb varies between individuals and is preferably measured for each patient. Typically, the haemoglobin levels range from 13.5-18 g/dL in adult males, 12-15 g/dL in adult females, and 11-16 g/dL in children. The total concentration of oxygen bound to haemoglobin may therefore be proportional to the oxygen saturation and the concentration of haemoglobin in the blood.

The partial pressure of oxygen pᵥO₂ / pₐO₂ may be calculated by using a relation referred to as Henrys' Law. At body temperature, about 0.0033 mL of oxygen is known to dissolve in 1 mL of blood plasma per mmHg of partial pressure of oxygen.

The above relations can be used to determine the concentration of oxygen in the blood post-gas exchange (CₐO₂) as well as an in the blood pre-gas exchange (CₐO₂). This allows for the oxygen uptake V_{b}O₂ in the blood (in terms of, e.g., L/min) to be determined by comparing the concentration of oxygen in the oxygen-rich blood with the concentration of oxygen in the blood pre-gas exchange (as indicated in equation 2 above) using the following relation: ${V}_{b} {O}_{2} = {Q}_{b} ⋅ 10 \frac{dL}{L} \left(1.34\frac{mL}{g}⋅Hb⋅\frac{{S}_{a} {O}_{2} - {S}_{v} {O}_{2}}{100}+0.0033\frac{mL}{dL ⋅ mmHg}\left({p}_{a}{O}_{2}-{p}_{v}{O}_{2}\right)\right)$ where SₐO₂ is the oxygen saturation in the blood post-gas exchange (%), SᵥO₂ is the oxygen saturation in the blood pre-gas exchange (%), pₐO₂ is the partial pressure of oxygen in the blood post-gas exchange (mmHg), and pᵥO₂ is the partial pressure of oxygen in the blood pre-gas exchange (mmHg).

It may be assumed that a major part of the oxygen supplied to the gas exchange device 110 is either taken up by the blood or passed as excess oxygen to the exhaust gas flow. Typically, the oxygen concentration of the inlet gas is adjusted based on the patient's needs and the type of heart/lung support provided. Oxygen concentration may thus range from about 21% to 100%. In case the inlet gas comprises 100% oxygen, the flow rate of oxygen VₛO₂ corresponds to the total flow rate Qₛ of the inlet gas. After the gas exchange has taken place, the oxygen concentration FₑO₂ in the exhaust gas (or 'spent' sweep gas) may still be relatively high, such as about 95% or more. It will however be appreciated that the oxygen concentration FₑO₂ in the exhaust gas may vary depending on several factors, such as the gas exchange efficiency between the sweep gas and the circulating blood, the flow rate Q_{b} of blood, the concentration of oxygen FₛO₂ in the sweep gas, the saturation of oxygen in the oxygen-poor blood, and the concentration of haemoglobin Hb.

The difference between the CO₂ flow rate V_{g}CO₂ transferred to the gas flow during the gas exchange and the reference flow rate V_{ref}CO₂ may then be compared to retrieve S150 information about the performance of the gas exchange device 110. It may for example be determined that the gas exchange device needs to be replaced, should the difference or quotient between V_{g}CO₂ and V_{ref}COₛ exceed a predetermined limit.

In some examples, the rate and magnitude of any changes in the CO₂ flow rate V_{g}CO₂ over time - referred to as the trend - may be monitored to assess whether the gas exchange device is functioning correctly or malfunctioning. A relatively stable CO₂ flow rate V_{g}CO₂ may indicate that the gas exchange device is functioning correctly. If issues are still present, their origin likely lies elsewhere, allowing for the exclusion of the gas exchange device as the source of the problem. Further, a pronounced trend in CO₂ flow rate V_{g}CO₂, even when it remains within acceptable limits, can be indicative of underlying issues that may not be immediately apparent from static measurements alone. For instance, a rapid decrease in in CO₂ flow rate V_{g}CO₂ might suggest evolving deterioration in gas transfer efficiency that require prompt attention to prevent further deterioration.

As mentioned above, the reference flow rate V_{ref}CO₂ may be determined by means of a model, which is designed to describe the relationship between a set of variables and constants. The mathematical framework may vary depending on the physical properties of the system, the range of interest, and the availability of data. However, in general, the model may be expressed as a mathematical function: ${V}_{ref} {CO}_{2} = f \left({x}_{1},{x}_{2},…,{x}_{n};{a}_{1},{a}_{2},…,{a}_{m}\right)$ where *x₁, x₂, ...xₙ* are the input or variables, *f (*·*)* represents the functional relationship between the input and the output, and *a₁, a₂,* ..., *aₘ* are constants or weights that adjust the function's behaviour to best fit the data or theory being applied. The constants may be determined through fitting the model to experimental data. Once set, they may remain constant for a particular type or design of the gas exchange device 110. The set of variables *x₁, x₂, ...xₙ* may vary between different examples of the present disclosure. Typically, the set of variables include one or more of the following:
- Q_{b}:: flow rate of blood passing through the gas exchange device
- Qₛ:: flow rate of gas flow supplied to the gas exchange device
- FₛCO₂:: concentration of CO₂ in the inlet gas
- pᵥCO₂:: partial pressure of CO₂ in blood entering oxygenator
- Hb:: haemoglobin concentration in the blood
- pH:: pH of the blood
The flow rates of blood Q_{b} and gas flow Qₛ have in some cases been observed to have the greatest influence on the transfer of CO₂ from the blood to the gas flow. Therefore, the model may be a function of those two variables: ${V}_{ref} {CO}_{2} = f \left({Q}_{b}, {Q}_{s}\right)$ Furthermore, in case the inlet gas comprises CO₂, this may be accounted for in the model: ${V}_{ref} {CO}_{2} = f \left({Q}_{b},{Q}_{s},{F}_{s}{CO}_{2}\right)$ In some examples, the model may be further improved by taking the partial pressure of carbon dioxide pᵥCO₂ of the blood entering the gas exchange device into account: ${V}_{ref} {CO}_{2} = f \left({Q}_{b},{Q}_{s},{F}_{s}{CO}_{2},{p}_{v}{CO}_{2}\right)$ The gas transfer efficiency may also be affected by the pH and/or the Hb . Hence, in some examples, the model takes this variable into account: ${V}_{ref} {CO}_{2} = f \left({Q}_{b},{Q}_{s},{F}_{s}{CO}_{2},{p}_{v}{CO}_{2},Hb,pH\right)$ It should be noted that in certain models, one or more variables such as pH, Hb, FₛCO₂, and pᵥCO₂ may be omitted. In particular, FₛCO₂ may be omitted when the inlet gas is devoid of CO₂.

Various types of models may be used, depending on the physical properties of the system, the desired accuracy, and the availability of data. The model may, for example, be a linear model expressing a direct proportionality between the reference flow rate VᵣₑₜCO₂ and one or more of the variables outlined above, or a polynomial model including terms of higher powers of one or more of the variables: $\begin{matrix}{V}_{ref} {CO}_{2} \left({Q}_{b},{Q}_{s},{F}_{s}{CO}_{2},Hb\right) = {a}_{1} + {a}_{2} {Q}_{b} + {a}_{3} {Q}_{s} + {a}_{4} {F}_{s} {CO}_{2} + {a}_{5} Hb + {a}_{6} {Q}_{b} ⋅ {Q}_{b} + \\ {a}_{7} {Q}_{b} ⋅ {Q}_{s} + {a}_{8} {F}_{s} {CO}_{2} ⋅ {F}_{s} {CO}_{2} + {a}_{9} Hb ⋅ Hb + {a}_{10} {Q}_{b} ⋅ {Q}_{s} + {a}_{11} {Q}_{b} {F}_{s} ⋅ {CO}_{2} + {a}_{12} {Q}_{b} ⋅ \\ Hb + {a}_{13} {Q}_{s} ⋅ {F}_{s} {CO}_{2} + {a}_{14} {Q}_{s} ⋅ {H}_{b} + {a}_{15} {F}_{s} {CO}_{2} ⋅ Hb\end{matrix}$ Higher order polynomial models, such as 6^{th} order polynomials, are also possible and may be applied in a similar way as outlined in example equation 13 above.

Sigmoid functions, such as the logistic function, may also be employed. Sigmoid functions typically have an 'S'-shaped curve, which may be suitable for modelling the gas transfer efficiency of a gas exchange device. An example comprising 4 parameters may be as follows: $f \left({x}_{1}, {x}_{2}, {x}_{3}, {x}_{4}\right) = \frac{a}{\left(1+{e}^{- {c}_{1} \left({x}_{1}-{d}_{1}\right)}\right) ⋅ \left(1+{e}^{- {c}_{2} \left({x}_{2}-{d}_{2}\right)}\right) ⋅ \left(1+{e}^{- {c}_{3} \left({x}_{3}-{d}_{3}\right)}\right) ⋅ \left(1+{e}^{- {c}_{4} \left({x}_{4}-{d}_{4}\right)}\right)} + b$ where *x₁* to *x₄* are Q_{b}, Qₛ, pᵥCO₂, and Hb, respectively, and a, b, and c₁ to c₄ are constants. Other examples of sigmoid functions include the hyperbolic tangent, arctangent, Gudermannian, error, generalised logistic, and smoothstep functions.

A regression method can be used to establish the relationship between the reference CO₂ flow rate (V_{ref}CO₂) and one or more variables. This includes the least square method, which minimised the sum of the squares of the differences between observed and predicted CO₂ flow rates are minimised. Other methods, such as Gaussian regression and ridge regression, are also possible. Additionally, V_{ref}CO₂ can be estimated using various machine learning models, ranging from regression trees to neural networks.

The constants in the model may be derived from observational data of the system, ideally covering a broad range of conditions to accurately reflect the system's behaviour under different scenarios. These data could be gathered from instances of CO₂ release to the gas flow in new, undeteriorated gas exchange devices 110 of the same model or design. Data collection may occur when replacing an old gas exchange device 110 with a new one or during dedicated test runs. It may be preferable to have data that include CO₂ flow rates transferred to the gas flow for diverse values of one or more of the variables, such as blood flow rate Q_{b}, inlet gas flow rate Qₛ, CO₂ concentration in the inlet gas FₛCO₂, partial pressure of carbon dioxide pᵥCO₂ in blood entering the gas exchange device 110, haemoglobin concentration Hb, and pH.

Various sensors may be employed to measure the oxygen concentration in the inlet gas and/or the exhaust gas, as readily understood by a person of ordinary skill in the art. However, it has been observed that condensation of water vapour in the exhaust gas pose challenges, as the water may interfere with the sensor and affect the relative concentration of other gases, including oxygen. This may in some examples be addressed by reducing the temperature of the exhaust gas to allow water vapour to condensate in a more controlled way before the sensor is exposed to the gas. The exhaust gas may be cooled to ambient room temperature or to even lower temperatures to further reduce the humidity of the exhaust gas. In further examples, the sensor may be heated directly (direct on or adjacent the sensor chip) or indirectly (whole sensor assembly/tube) to reduce the risk of condensed water interfering with the operation of the sensor. Additionally, or alternatively, the exhaust gas may be brought to pass through a device allowing water vapour to be removed from the gas. An example of such a device is the Nafion tube. Combinations are also possible, in which the exhaust gas is cooled, and the sensor heated to further reduce the risk of condensation.

As for the blood gas measurements, oxygen sensors may be employed to measure the oxygen content in the blood, such as oxygen saturation and partial pressure of oxygen in the blood post-gas exchange as well as of the blood pre-gas exchange. Sensors such as optodes or Clark electrodes may be used to measure the partial pressure of oxygen in the blood post gas exchange and pre-gas exchange, respectively. Each of the oxygen sensor 122 at the inlet side of the gas exchange device 110 and the oxygen sensor 123 at the outlet side of the gas exchange device 110 may hence comprise a respective sensor for measuring saturation and partial pressure, and in some examples also the haemoglobin concentration Hb in the blood.

The CO₂ concentration in the gas flow, the inlet gas, and/or the outlet gas, may be retrieved from one or more CO₂ sensors, such as the CO₂ sensor 125 arranged at the inlet of the gas exchange device 110 and the CO₂ sensor 126 arranged at the outlet of the gas exchange device 110. Examples of CO₂ sensors include, but are not limited to, infrared sensors correlating the level of absorption of infrared light by carbon dioxide molecules to the concentration of carbon dioxide molecules in the exhaust gas. Infrared sensors and Severinghaus electrodes may also be used to measure the partial pressures of carbon dioxide pᵥCO₂ / pₐCO₂ in the blood.

It will be appreciated that any of the sensors discussed herein may be integrated in the gas exchange device 110, i.e., permanently or replaceably installed at, for example, an outlet of the gas exchange device 110. However, in other examples, one or more of the sensors may be a separate unit configured to perform measurements on the gas flow or the blood flow, for example. In yet further examples, one or more of the sensors may be integrated into a gas supply module or an exhaust gas module as discussed above.

The system 100 of the present disclosure may generally comprise one or more control units 140, or processors, and one or more non-transitory computer-readable media storing first computer executable instructions that, when executed by the one or more processors, cause the system 100 to perform at least parts of the actions shown in figures 3 and 4 and described above.

Generally, the system 100 may comprise circuitry which is configured to implement (using one or more non-transitory computer-readable media) the functionality described herein. Suitable processors for the execution of a program of instructions include, by way of example, both general and special purpose microprocessors, and the sole processor or one of multiple processors or cores, of any kind of computer. The processors can be supplemented by, or incorporated in, ASICs (application-specific integrated circuits). Those skilled in the art will understand that the above-described exemplary embodiments may be implemented in any suitable software, hardware, or firmware configuration or combination thereof. An exemplary hardware platform for implementing the exemplary embodiments may include, for example, an Intel x86 based platform with compatible operating system, a Windows OS, a Linux-based OS, a Mac platform and MAC OS, a mobile device having an operating system such as iOS, Android, etc. In a further example, the exemplary embodiments of the above-described method may be embodied as a program containing lines of code stored on a non-transitory computer readable storage medium that, when compiled, may be executed on a processor or microprocessor.

The above embodiments are to be understood as illustrative examples of the invention. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

## Claims

1. A system (100) comprising:
a gas exchange device (110) for transferring blood gases between blood and a gas flow passing through the gas exchange device, the gas flow entering the gas exchange device as an inlet gas and exiting the gas exchange device as an exhaust gas;
a gas supply module configured to generate a first signal indicating a flow rate of carbon dioxide VₛCO₂ in the inlet gas;
an exhaust gas module configured to generate a second signal indicating a flow rate of carbon dioxide VₑCO₂ in the exhaust gas; and
a control unit (140) configured to receive the first and second signals, and to:
determine a flow rate of carbon dioxide V_{g}CO₂ transferred to the gas flow based on a difference between the flow rate of carbon dioxide VₑCO₂ in the exhaust gas and the flow rate of carbon dioxide VₛCO₂ in the inlet gas; and
determine a performance of the gas exchange device based on the flow rate of carbon dioxide V_{g}CO₂ transferred to the gas flow and a reference flow rate V_{ref}CO₂;
wherein the reference flow rate is based on at least one of: a flow rate of blood through the gas exchange device Q_{b}, and a flow rate of the gas flow Qₛ.

2. The system according to claim 1, further comprising:
an oxygen sensor arrangement (122, 123, 124) configured to generate sensor data indicating an oxygen concentration in the blood pre-gas exchange CᵥO₂, an oxygen concentration CₐO₂ in blood post-gas exchange, and an oxygen concentration FₑO₂ in the exhaust gas;
wherein the control unit is configured to:
determine an oxygen uptake V_{b}O₂ in the blood based on the flow rate of blood Q_{b} and a difference between the oxygen concentration CₐO₂ in the blood post-gas exchange and the oxygen concentration CᵥO₂ in the blood pre-gas exchange;
determine a flow rate of oxygen VₑO₂ in the exhaust gas based on a difference between a flow rate of oxygen in the inlet gas VₛO₂ and the oxygen uptake V_{b}O₂ in the blood; and
determine the total flow rate of the exhaust gas Qₑ based on the flow rate of oxygen VₑO₂ in the exhaust gas and the oxygen concentration FₑO₂ in the exhaust gas.

3. The system according to claim 2, further comprising a blood gas sensor arrangement (125, 126) configured to generate sensor data indicating the partial pressure of carbon dioxide pᵥCO₂ in the blood pre-gas exchange and a partial pressure of carbon dioxide pₐCO₂ in the blood post-gas exchange.

4. The system according to claim 3, wherein the control unit is configured to determine a blood gas-based flow rate of carbon dioxide V_{b}CO₂ transferred to the gas flow based at least in part on at least one of the flow rate of blood Q_{b}, the partial pressure of carbon dioxide pᵥCO₂ in the blood pre-gas exchange, the partial pressure of carbon dioxide pₐCO₂ in the blood post-gas exchange, the haemoglobin concentration Hb in the blood, the oxygen saturation pre- and post-gas exchange, and the pH of the blood pre- and post-gas exchange.

5. The system according to claim 4, wherein the control unit is further configured to compare the blood gas-based flow rate of carbon dioxide V_{b}CO₂ transferred to the gas flow with a gas flow-based flow rate of carbon dioxide V_{g}CO₂ transferred to the gas flow, wherein the gas flow-based flow rate V_{g}CO₂ is determined based on the flow rate of carbon dioxide VₑCO₂ in the exhaust gas and the flow rate of carbon dioxide VₛCO₂ in the inlet gas.

6. The system according to claim 5, wherein the control unit is further configured to determine, based on the comparison, that the blood gas-based flow rate of carbon dioxide V_{b}CO₂ corresponds to the gas flow-based flow rate of carbon dioxide V_{g}CO₂.

7. The system according to claim 5, comprising determining an error state based on a difference between the blood gas-based flow rate of carbon dioxide V_{b}CO₂ and the gas flow-based flow rate of carbon dioxide V_{g}CO₂ exceeding a predetermined limit.

8. The system according to claim 5, wherein the control unit is further configured to determine the performance of the gas exchange device based on a comparison between the reference flow rate V_{ref}CO₂ and an average of the blood gas-based flow rate of carbon dioxide V_{b}CO₂ and the gas flow-based flow rate V_{g}CO₂.

9. The system according to claim 1, wherein the reference flow rate is based on at least one of the flow rate of blood through the gas exchange device Q_{b}, the flow rate of the gas flow Qₛ, a concentration of carbon dioxide FₛCO₂ in the inlet gas, a partial pressure pᵥCO₂ of carbon dioxide in blood pre-gas exchange, a haemoglobin concentration Hb in the blood, and a pH of the blood.

10. The system according to claim 1, wherein the reference flow rate is based on a combination of the flow rate of blood through the gas exchange device Q_{b}, the flow rate of the gas flow Qₛ, a concentration of carbon dioxide FₛCO₂ in the inlet gas, and a partial pressure pᵥCO₂ of carbon dioxide in blood pre-gas exchange.

11. The system according to claim 1, wherein the reference flow rate V_{ref}CO₂ is based on a model describing a flow rate of carbon dioxide transferred to the gas flow as a function of at least one of: the flow rate of blood through the gas exchange device Q_{b}, the flow rate of the gas flow Qₛ, the concentration of carbon dioxide FₛCO₂ in the inlet gas, the partial pressure pᵥCO₂ of carbon dioxide in blood pre-gas exchange, the haemoglobin concentration Hb in the blood, and the pH of the blood.

12. The system according to claim 11, wherein the model describes the flow rate of carbon dioxide transferred to the gas flow as a function of the flow rate of blood through the gas exchange device Qb and the flow rate of the gas flow Qs.

13. The system according to claim 1, wherein the model describes the reference flow rate V_{ref}CO₂ corresponding to a flow rate of carbon dioxide transferred to the gas flow by a fresh gas exchange device.

14. The system according to claim 13, wherein the model is based on experimental data retrieved from one or more fresh gas exchange devices.

15. The system according to claim 1, wherein the control unit is further configured to indicate that the gas exchange device needs to be replaced based on a comparison between the flow rate of carbon dioxide V_{g}CO₂ transferred to the gas flow and the reference flow rate V_{ref}CO₂.
